# EUROPÄISCHE PATENTSCHRIFT

(11) **EP 2 853 284 B1**
(45) Veröffentlichungstag und Bekanntmachung des Hinweises auf die Patenterteilung: **11.12.2019**
(21) Anmeldenummer: 14183053.9
(22) Anmeldetag: 01.09.2014
(51) Int. Cl.: A61M 25/01

(54) **Katheterisierungsvorrichtung mit einem ein auslenkbares Lenkende aufweisenden Katheter**
Catheterization device comprising a catheter, which has a deflectable steering end
Dispositif de cathétérisation doté d'un cathéter présentant une extrémité articulée extensible

(30) Priorität: 25.09.2013 US 201361881977 P
(43) Veröffentlichungstag der Anmeldung: 01.04.2015
(73) Patentinhaber: VascoMed GmbH, 79589 Binzen (DE)
(72) Erfinder: Maurice, Ingmar, 1018AK Amsterdam (NL)
(74) Vertreter: Galander, Marcus

(56) Entgegenhaltungen:
- US-A1- 2002 065 485

## Beschreibung

Die Erfindung betrifft eine Katheterisierungsvorrichtung mit einem ein auslenkbares Lenkende aufweisenden Katheter und einer Steuerungsvorrichtung zur Steuerung des Lenkendes, wobei die Steuerungsvorrichtung mit einer um eine Drehachse drehbar gelagerte Steuerungseinrichtung, die zumindest ein von der Drehachse beabstandet angeordnetes und bewegungsübertragend mit dem Lenkende verbundenes Führungselement aufweist, ausgebildet ist.

Katheterisierungsvorrichtungen der eingangs genannten Art sind allgemein bekannt und werden zur Untersuchung und Behandlung von Patienten, beispielsweise mit Herzkrankheiten, verwendet. Um den Katheter zum Beispiel bis zum Herzen führen zu und dort ausgewählte Bereich des Herzens untersuchen und/oder behandeln zu können, ist ein distales Ende des Katheters als das Lenkende ausgebildet. Von einem dem distalen Ende entlang des Katheters gegenüberliegenden proximalen Ende des Katheters kann das Lenkende gesteuert und insbesondere in wenigstens eine, zwei oder mehr als zwei unterschiedliche Richtungen geschwenkt werden. Zum Schwenken des Lenkendes ist beispielsweise eine Lenkdraht vom Lenkende bis zum proximalen Ende geführt und dort an der Steuerungseinrichtung fixiert. Wird die Steuerungseinrichtung um die Drehachse herum bewegt, so zieht sie am Lenkdraht und eine das Lenkende auszulenken suchende Kraft wird über den Lenkdraht zum Lenkende geleitet.

Das Dokument US 2002/0065485 A1 offenbart einen bi-direktional deflektierbaren Katheter mit einem Griff. Der Griff weist eine Steuereinrichtung zur Deflektion des Katheterschafts mittels eines Zugdrahts auf. Die Steuereinrichtung umfasst eine Vorrichtung zum Erzeugen einer Gegenkraft, die einer von dem Zugdraht bei der Deflektion des Schafts erzeugten Kraft entgegen wirkt.

Bei den bekannten Katheterisierungsvorrichtungen tritt jedoch das Problem auf, dass je weiter das Lenkende aus einer Ruheposition heraus geschwenkt werden soll, desto höher die hierfür benötigen Bedienkräfte werden. Da die Bedienkräfte meistens manuell von einem Bediener in die Steuerungseinrichtung eingeleitet werden und hohe Bedienkräfte durch den Bediener der Katheterisierungsvorrichtung nur für eine begrenzte Zeit aufgebracht werden können, ist es die Aufgabe der Erfindung, eine Katheterisierungsvorrichtung bereitzustellen, die einfach und insbesondere mit einem geringen Kraftaufwand bedienbar ist.

Die Aufgabe wird für die eingangs genannte Katheterisierungsvorrichtung durch einen Kraftspeicher gelöst, der eine Drehung der Steuerungseinrichtung unterstützende Hilfskraft bewirkt, wobei die Steuerungseinrichtung ein von der Drehachse und dem Führungselement beabstandet angeordnetes Kraftübertragungselement aufweist, dass kraftübertragend mit dem Kraftspeicher gekoppelt ist.

Es ist eine Katheterisierungsvorrichtung mit den Merkmalen nach Anspruch 1 bereitgestellt.

Durch den Kraftspeicher und die vom Kraftspeicher bereitgestellte Hilfskraft wird die zum Auslenken notwendige und manuell in die Steuerungseinrichtung einzuleitende Kraft verringert, so dass die Belastung des Bedieners der Katheterisierungsvorrichtung abnimmt und die Bedienung der Katheterisierungsvorrichtung leichter wird.

Die erfindungsgemäße Lösung kann durch verschiedene, jeweils für sich vorteilhafte, beliebig miteinander kombinierbare Ausgestaltungen weiter verbessert werden. Auf diese Ausgestaltungsformen und die mit Ihnen verbundenen Vorteile ist im Folgenden eingegangen, wobei die konstruktiven Maßnahmen und deren Wirkungen lediglich beispielhaft für einen Katheter mit nur einem Lenkdraht beschrieben sind. Selbstverständlich kann der Katheter mehrere und insbesondere zwei Lenkdrähte aufweisen und dessen Lenkende somit in mehr als eine Richtung durch die Steuerungsvorrichtung ausgelenkt werden.

In einer ersten vorteilhaften Ausgestaltungsform kann ein die Drehachse und das Kraftübertragungselement miteinander verbindender Radius senkrecht zu einem Wirkkraftvektor der Hilfskraft ausgerichtet sein, wobei eine Wirklänge des Wirkkraftvektors abhängig von der Auslenkung der Steuerungseinrichtung aus einer Ruhestellung ist. Das Kraftübertragungselement ist vorzugsweise starr mit dem Führungselement verbunden und bewegt sich bei der Bedienung der Steuerungsvorrichtung mit dem Führungselement um die Drehachse. Die Hilfskraft kann also je nach Betrag des Auslenkens des Katheters unterschiedlich vordefiniert beziehungsweise vorab eingestellt sein. Eine manuelle Einstellung der Hilfskraft beziehungsweise der Wirklänge des Wirkkraftvektors während der Katheterisierung ist also nicht notwendig.

Um zu vermeiden, dass sich die Steuerungseinrichtung selbsttätig aus ihrer Ruhestellung bewegt, kann die Wirklänge in der Ruhestellung minimal sein. Insbesondere wenn die Wirklänge Null ist, sucht die Hilfskraft die Steuerungseinrichtung nicht aus der Ruhestellung zu bewegen, so dass die Ruhestellung der Steuerungseinrichtung zumindest instabil oder sogar stabil ist.

Die Wirklänge steigt mit der Auslenkung der Steuerungseinrichtung aus der Ruhestellung und vorzugsweise proportional dazu an. Insbesondere bei vergleichsweise kleinen Auslenkungen ist der Anstieg proportional. Da die Wirklänge des Wirkkraftvektors abhängig von der Größe der Auslenkung der Steuerungseinrichtung aus ihrer Ruhestellung ist, kann die Hilfskraft mit der Auslenkung der Steuerungseinrichtung zunehmen, so dass die manuell in die Steuerungseinrichtung einzuleitende Bedienkraft im Wesentlichen für alle möglichen Auslenkungen des Lenkendes konstant ist oder sich nur wenig ändert. Insgesamt können sich die auf das Kraftübertragungselement einwirkenden Kräfte im Wesentlichen ausgleichen, so dass das Ende des Katheters in einer beliebigen ausgelenkten Position verbleibt, selbst wenn der Bediener der Katheterisierungsvorrichtung das Bedienelement nicht in Position hält.

Der Wirkkraftvektor ist beispielsweise eine Komponente der vom Kraftspeicher an das Kraftübertragungselement eingeleiteten Kraft, wobei die in das Kraftübertragungselement eingeleitete Kraft in der Ruhestellung der Steuerungseinrichtung vorzugsweise auf die Drehachse gerichtet ist. Da die Wirklänge des Wirkkraftvektors in diesem Fall automatisch Null ist, ist die Hilfskraft in der Ruhestellung ebenfalls Null und es sind keine die Hilfskraft in der Ruhestellung begrenzenden oder kompensierenden Maßnahmen notwendig, um die Steuerungseinrichtung in der Ruhestellung zu halten.

Die Steuerungsvorrichtung kann eine Halteeinrichtung für den Kraftspeicher aufweisen, an der der Kraftspeicher zumindest in Richtung auf die Drehachse unverschieblich gehalten ist. So kann die Halteeinrichtung ortsfest in der Steuerungsvorrichtung vorgesehen sein und ein vom mit dem Kraftübertragungselement verbundenen Übertragungsende des Kraftspeichers gegenüberliegendes Halteende des Kraftspeichers ortsfest halten. Alternativ kann das sich an der Halteeinrichtung abstützende Halteende im Wesentlichen senkrecht von der in das Kraftübertragungselement eingeleiteten Kraft beweglich gehalten sein. Bei einem beweglich gehaltenen Halteende kann die Wirkkraftlänge bei einer Auslenkung der Steuerungseinrichtung um 90° um die Drehachse maximal sein und somit den Bediener optimal unterstützen.

Die durch den Kraftspeicher in der Ruhestellung auf das Kraftübertragungselement gerichtete Kraft kann eine Zugkraft sein. Dabei ist das Kraftübertragungselement vorzugsweise in der Ruhestellung von der Halteeinrichtung aus hinter der Drehachse angeordnet. Hierdurch ist der Aufbau der Steuerungsvorrichtung einfach und kompakt möglich.

Um die Steuerungsvorrichtung noch einfacher und kompakter ausgestalten zu können, kann die durch den Kraftspeicher in der Ruhestellung auf das Kraftübertragungselement gerichtete Kraft eine Druckkraft sein. In diesem Fall ist das Kraftübertragungselement in der Ruhestellung vorzugsweise zwischen der Drehachse und der Halteeinrichtung angeordnet. Der die Druckkraft hervorrufende Kraftspeicher kann somit kleiner und kompakter ausgestaltet sein, als der die Zugkraft erzeugende Kraftspeicher.

Der Kraftspeicher kann beispielsweise ein pneumatischer Kraftspeicher sein, der die Zu- oder die Druckkraft bereitstellt. Vorzugsweise stellt der Kraftspeicher eine elastische und insbesondere eine federelastische Kraft zur Unterstützung der Bedienung der Steuerungsvorrichtung bereit. Ein besonders einfacher und dabei sehr zuverlässiger Kraftspeicher ist eine Zug- oder eine Druckfeder, beispielsweise eine Spiralfeder, die zudem auch geringe Kosten verursacht.

Gemäß einer weiteren vorteilhaften Ausgestaltungsform kann die Steuerungsvorrichtung ein zahnstangenförmiges Bedienelement aufweisen, dessen Zähne bewegungsübertragend auf die Steuerungseinrichtung einwirken. Die Steuerungseinrichtung kann zur bewegungsübertragenden Verbindung mit der Zahnstange an ihrem Umfang wenigstens einen Zahn aufweisen, der in wenigstens zwei Zähne der Zahnstange formschlüssig eingreift. Beispielsweise kann die Steuerungseinrichtung als ein Zahnrad ausgebildet sein. Ein Verschieben des zahnstangenförmigen Bedienelements rotiert die Steuerungseinrichtung. Eine derartige Steuerungsvorrichtung ist kompakt ausgebildet, da beispielsweise ein aus einem Gehäuse der Steuerungsvorrichtung heraus ragender Bedienhebel nicht nötigt ist.

Die Katheterisierungsvorrichtung kann ein Getriebe aufweisen, das den Kraftspeicher Kraft übertragend mit dem Kraftübertragungselement verbindet. Die Höhe der vom Kraftübertragungselement aufgenommenen Kraft kann durch das Getriebe angepasst sein oder speziell an die aktuellen Anforderungen angepasst werden. Insbesondere bei vergleichsweise großen Auslenkungen kann es sein, dass die Hilfskraft nicht mehr proportional mit für das Auslenken des Lenkendes benötigten Kraft ansteigt. Zur Anpassung der Hilfskraft kann die Katheterisierungsvorrichtung ein die Hilfskraft übertragendes Getriebe aufweisen. Das Getriebe kann so ausgestaltet sein, dass die maximal mögliche Auslenkung des Lenkendes erreicht wird, selbst wenn das Bedienelement seine maximal mögliche Auslenkung nicht vollständig erreicht. Die benötigte Auslenkung des Bedienelements kann also geringer sein. Insgesamt können sich die auf das Kraftübertragungselement einwirkenden Kräfte im Wesentlichen ausgleichen, so dass das Ende des Katheters in einer beliebigen ausgelenkten Position verbleibt, selbst wenn der Bediener der Katheterisierungsvorrichtung das Bedienelement nicht in Position hält. Vorzugsweise ist das Getriebe so ausgestaltet, dass die maximal mögliche Auslenkung des Lenkendes erreicht wird, bevor das Bedienelement seine maximal mögliche Auslenkung vollständig erreicht. Hierdurch ist gewährleistet, dass die zum Auslenken des Lenkendes benötigte Kraft sich auch bei einer großen und insbesondere bis zur maximal möglichen Auslenkung des Bedienelementes annähernd linear mit dem Grad der Auslenkung ändert.

Die Katheterisierungsvorrichtung und insbesondere der Katheter kann den wenigstens einen Lenkdraht aufweisen, mit dem eine das Lenkende auslenkende Kraft von der Steuerungseinrichtung an das Lenkende übertragen wird. Der Lenkdraht ist vorzugsweise an einer Umfangsfläche der Steuerungseinrichtung geführt.

Eine Länge eines um die Drehachse schwenkbaren Lastarms der Steuerungseinrichtung kann abhängig von der Auslenkung der Steuerungseinrichtung sein. Der Lastarm kann sich beispielsweise zwischen der Drehachse und einer Umfangsfläche der Steuerungseinrichtung der scheibenförmigen Basis erstrecken. An der Umfangsfläche kann der Lenkdraht anliegen. Die Umfangsfläche kann unterschiedliche und beispielsweise sich in ihrem Verlauf kontinuierliche Abstände zur Drehachse aufweise, wodurch die Kraft zum Auslenken des Lenkendes an den Grad der Auslenkung anpassbar sein kann. Einfacher herzustellen ist die Katheterisierungsvorrichtung jedoch, wenn die Länge des Lastarms unabhängig von der Auslenkung der Steuerungseinrichtung aus der Ruhestellung ist. Hierzu weist die Umfangsfläche vorzugsweise einen konstanten Abstand zur Drehachse auf.

Die scheibenförmige Basis der Steuerungseinrichtung kann zumindest teilweise und insbesondere in Führungsabschnitten, an denen der Lenkdraht abhängig von der Auslenkung der Steuerungseinrichtung anliegt, kreisförmig ausgeformt sein.

Die Hilfskraft ist vorzugsweise bemessen, das Lenkende in einer ausgelenkten Position zu halten. Insbesondere wenn keine äußeren Kräfte auf das Lenkende einwirken, kann die Hilfskraft, womöglich zusammen mit innerhalb der Katheterisierungsvorrichtung wirkenden Reibkräften, ein ungewolltes Schwenken des Lenkendes aus der eingestellten Schwenkposition heraus zu verhindern. Treten äußere Kräfte auf, die auf das Lenkende einwirken und es auszulenken suchen, kann die Hilfskraft, wieder womöglich zusammen mit innerhalb der Katheterisierungsvorrichtung wirkenden Reibkräften, so eingestellt oder einstellbar sein, dass das Lenkende die eingestellte Schwenkposition trotz der äußeren Kräfte beibehält.

Die in den Ausführungsformen dargestellte und beschriebene Steuerungsvorrichtung kann auch ohne den oder die Katheter oder als ein Bausatz mit mehreren Kathetern, von denen zumindest einige nicht bewegungsübertragend mit der Steuerungseinrichtung verbunden sind, bereitgestellt sein

Im Folgenden ist die Erfindung beispielhaft anhand von Ausführungsbeispielen mit Bezug auf die Zeichnungen erläutert. Die unterschiedlichen Merkmale der Ausführungsformen können dabei unabhängig voneinander kombiniert werden, wie es bei den einzelnen vorteilhaften Ausgestaltungen bereits dargelegt wurde.

Es zeigen:
- Fig. 1 bis 4: schematisch verschiedene Ausführungsbeispiele der erfindungsgemäßen Katheterisierungsvorrichtung;
- Fig. 5: ein weiteres Ausführungsbeispiel der erfindungsgemäßen Katheterisierungsvorrichtung schematisch in einer Aufsicht; und
- Fig. 6: ein weiteres Ausführungsbeispiel der erfindungsgemäßen Katheterisierungsvorrichtung in einer schematischen Perspektivansicht.

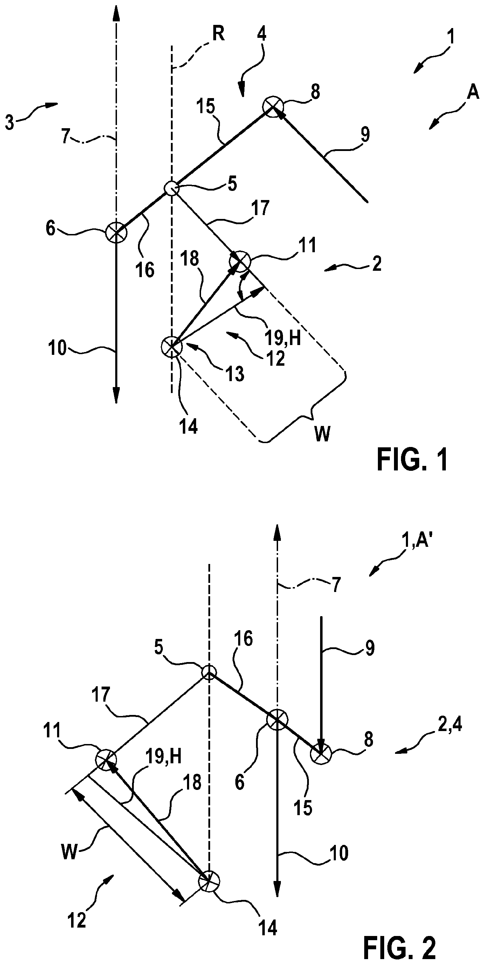

Figur 1 zeigt die Katheterisierungsvorrichtung 1 schematisch mit einer Steuerungsvorrichtung 2 und einem Katheter 3.

Die Steuerungsvorrichtung 1 ist mit einer Steuerungseinrichtung 4 dargestellt. Die Steuerungseinrichtung 4 ist in der Figur 1 stark schematisiert und lediglich mit Hebeln, Kraftaufbeziehungsweise Kraftabgabeelementen und einer Drehachse 5 gezeigt. Um die senkrecht zur Zeichenebene verlaufende Drehachse 5 ist die Steuerungseinrichtung 4 in der Steuerungsvorrichtung 2 drehbar gehalten. Die Drehachse 5 wandelt auf die Steuerungseinrichtung 4 einwirkende Kräfte in Drehmomente und gibt insbesondere Bedienkräfte an das Lenkende des Katheters 3 weiter.

Die Steuerungseinrichtung 4 weist ein Führungselement 6 auf, dass bewegungsübertragend mit dem Lenkende verbindbar ist. Im Ausführungsbeispiel der Figur 1 ist das Führungselement 6 mit einem Lenkdraht 7 des Katheters 3 bewegungsübertragend verbunden. Der Lenkdraht 7 kann am Führungselement 6 fixiert und beispielsweise durch eine Schraubverbindung verklemmt sein. Ferner weist die Steuerungseinrichtung 4 ein Bedienelement 8 auf, in das ein Bediener der Katheterisierungsvorrichtung eine Bedienkraft 9 zum auslenken des Lenkendes einleiten kann. Die Steuerungseinrichtung 4 und die Drehachse 5 wandeln die Bedienkraft 9 in eine Auslenkkraft 10, die beispielsweise als eine Zugkraft ausgebildet ist und den Lenkdraht zum auslenken des Lenkendes aus einer Ruheposition auszulenken sucht.

Die Steuerungsvorrichtung 2 ist ferner mit einem Kraftübertragungselement 11 ausgebildet, das beabstandet von der Drehachse 5 und dem Führungselement 6 durch die Steuerungseinrichtung 4 ausgebildet ist. Das Führungselement 6, das Bedienelement 8 und das Kraftübertragungselement 11 sind vorzugsweise bewegungsübertragend und vorzugsweise starr miteinander verbunden.

Darüber hinaus ist die Steuerungsvorrichtung 2 mit einem Kraftspeicher 12 gezeigt, dessen Halteende 13 an einer Halteeinrichtung 14 befestigt ist. Die Halteeinrichtung 14 ist vorzugsweise zumindest in Richtung auf die Drehachse 5 unverschieblich in der Steuerungsvorrichtung 2 aufgenommen.

Eine Ruheposition R der Steuerungsvorrichtung 2 ist durch eine die Drehachse 5 schneidende gestrichelte Linie angedeutet. Entspricht eine Betriebsstellung der Steuerungseinrichtung 4 der Ruheposition R befindet sich die Steuerungseinrichtung 4 also in Ihrer Ruhestellung, so ist das Lenkende des Katheters 3 nicht ausgelenkt. Beispielsweise ist ein die Drehachse 5 mit dem Bedienelement 8 verbindender Kraftarm 15 der Steuerungseinrichtung 4 in der Ruheposition R senkrecht zu der gestrichelten Linie ausgerichtet. Auch ein Lastarm 16, der sich von der Drehachse 5 zum Führungselement 6 erstreckt, kann in der Ruhestellung R senkrecht zu der gestrichelten Linie verlaufen. Ein von der Drehachse 5 zum Kraftübertragungselement 11 verlaufender Radius 17 ist in der Ruheposition R parallel zu der gestrichelten Linie ausgerichtet. Insbesondere weist der Radius 17 in der Ruheposition R von der Drehachse 5 zur Halteeinrichtung 14.

Die Steuerungseinrichtung 4 ist in der Figur 1 nicht in ihrer Ruhestellung, sondern in einer Auslenkstellung A dargestellt, durch die in das Bedienelement 8 eingeleitete Bedienkraft 9 ist die Steuerungseinrichtung 4 entgegen dem Uhrzeigersinn aus ihrer Ruhestellung ausgelenkt. Der Lenkdraht 7 ist im Vergleich zur Ruhestellung in Richtung der Auslenkkraft 10 verschoben. Das in der Ruhestellung zwischen der Drehachse 5 und der Halteeinrichtung 14 angeordnete Kraftübertragungselement 11 ist ebenfalls entgegen dem Uhrzeigersinn ausgelenkt.

Eine Kraft des Kraftspeichers 12 wirkt mechanisch auf das Kraftübertragungselement 11 ein, wobei die durch den Kraftspeicher 12 abgegebene Kraft als ein Kraftvektor 18 dargestellt ist, der von der Halteeinrichtung 14 zum Kraftübertragungselement 11 weist. Der Kraftspeicher 12 speichert also eine Druckkraft, die auf das Kraftübertragungselement 11 einwirkt und es entgegen dem Uhrzeigersinn weiter aus der Ruhestellung zu drücken sucht.

Die durch den Kraftvektor 18 dargestellte Druckkraft des Kraftspeichers 12 drückt das Kraftübertragungselement 11 jedoch nicht vollständig weiter aus der Ruhestellung. Lediglich eine Komponente des Kraftvektor 18 und insbesondere eine als ein Wirkkraftvektor 19 dargestellte und senkrecht zum Radius 17 verlaufende Komponente des Wirkkraftvektors 18 sucht das Kraftübertragungselement 11 weiter auszulenken. Eine durch den Betrag des Wirkkraftvektors 19 bestimmte Wirklänge W ändert sich abhängig von der Betriebsstellung der Steuerungseinrichtung 4. Ist die Steuerungseinrichtung 4 in der Ruhestellung ausgerichtet, so ist die Wirklänge W minimal und insbesondere Null, da der Kraftvektor 18 von der Halteeinrichtung 14 auf die Drehachse 5 zuweist. Da der Radius 17 und der Kraftvektor 18 in der Ruhestellung der Steuerungseinrichtung 4 entgegengerichtet ausgerichtet sind, ist der senkrecht zum Radius 17 verlaufende Wirkkraftvektor 19 des Kraftvektors 18 gleich Null. Die durch den Kraftspeicher 12 in das Kraftübertragungselement 11 eingeleitete Kraft bewirkt in der Ruhestellung der Steuerungseinrichtung 4 also nicht, dass die Steuerungseinrichtung 4 aus der Ruhestellung R wird.

Wird die Steuerungseinrichtung 4 aus ihrer Ruhestellung heraus bewegt und beispielsweise entgegen dem Uhrzeigersinn um die Drehachse 5 verdreht, so steigt die Wirklänge W des Wirkkraftvektors 19 proportional zur Auslenkung der Steuerungseinrichtung 4 aus der Ruhestellung an. Die Wirklänge W des Wirkkraftvektors 19 ist proportional zu einer Hilfskraft H, die den Bediener bei der Betätigung des Lenkendes unterstützt.

Figur 2 zeigt ein weiteres Ausführungsbeispiel der Erfindung, wobei für Elemente, die in Funktion und /oder Aufbau den Elementen des Ausführungsbeispiels der Figur 1 entsprechen, dieselben Bezugszeichen verwendet wurde. Der Kürze halber ist lediglich auf die Unterschiede zum Ausführungsbeispiel der Figur 1 eingegangen.

Die Katheterisierungsvorrichtung 1 ist im Ausführungsbeispiel der Figur 2 ebenfalls in einer Auslenkstellung A' dargestellt und weist alle Elemente der Katheterisierungsvorrichtung 1 des Ausführungsbeispiels 1 auf. Im Unterschied dazu sind der Kraftarm 15 und der Lastarm 16 jedoch nicht voneinander weg weisend auf unterschiedlichen Seiten der Drehachse 5 angeordnet. Wie im Ausführungsbeispiel der Figur 2 gezeigt, können der Kraftarm 15 und der Lastarm 16 auf der gleiche Seite der Drehachse 5 und insbesondere einander überlappend angeordnet sein, um einen kompakteren Aufbau der Steuerungsvorrichtung 2 zu ermöglichen.

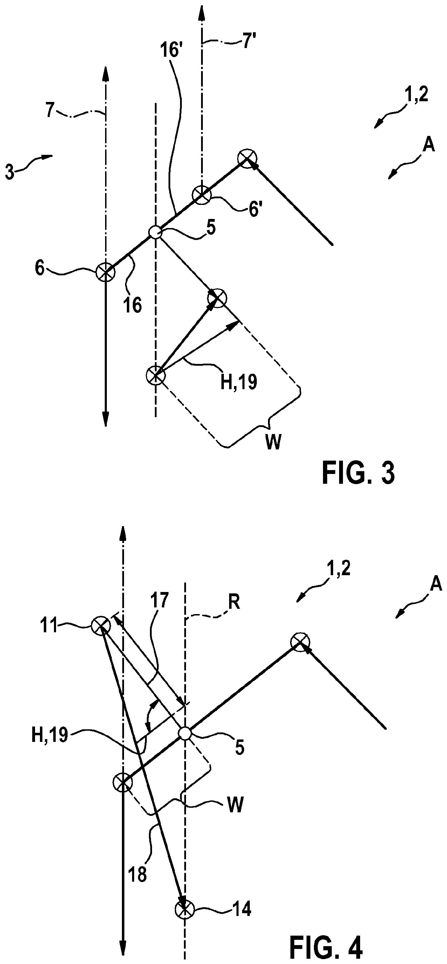

Figur 3 zeigt ein weiteres Ausführungsbeispiel der Erfindung, wobei für Elemente, die in Funktion und/oder Aufbau den Elementen der Ausführungsbeispiele der Figuren 1 und 2 entsprechen, dieselben Bezugszeichen verwendet sind. Der Kürze halber ist lediglich auf die Unterschiede zu den Ausführungsbeispielen der bisherigen Figuren eingegangen.

Der Katheter 3 des Ausführungsbeispiels der Figur 3 weist ein in unterschiedliche Richtungen auslenkbares Lenkende auf. Insbesondere ist das Lenkende des Katheters 3 in voneinander weg weisende Richtungen schwenkbar. Um das Lenkende des Katheters 3 in die beiden Richtungen schwenken zu können, ist der Katheter 3 mit zwei Lenkdrähten 7, 7' ausgerüstet. Der Lenkdraht 7 ist wie im Ausführungsbeispiel der Figur 1 am Führungselement 6 befestigt. Das Führungselement 6 ist über den Lastarm 16 mit der Drehachse 5 verbunden. Der Lenkdraht 7' ist an einem weiteren Führungselement 6' angebracht, wobei das Führungselement 6' durch einen weiteren Lastarm 16' mit der Drehachse 5 verbunden ist. Um das Lenkende in entgegengesetzte Richtungen schwenken zu können, weisen die Lastarme 16, 16' in unterschiedliche und insbesondere entgegengesetzte Richtungen von der Drehachse 5 weg. Die Drehachse 5 ist also vorzugsweise zwischen den Führungselementen 6, 6' angeordnet.

Figur 4 zeigt ein weiteres Ausführungsbeispiel der Erfindung, wobei für Elemente, die in Funktion und/oder Aufbau den Elementen der Ausführungsbeispiele der bisherigen Figuren entsprechen, dieselben Bezugszeichen verwendet sind. Der Kürze halber ist im Folgenden lediglich auf die Unterschiede zu den bisherigen Ausführungsbeispielen eingegangen.

Die Katheterisierungsvorrichtung 1 ist in der Figur 4 mit einer Steuerungsvorrichtung 2 dargestellt, deren Kraftübertragungselement 11 von der Halteeinrichtung 14 aus gesehen hinter der Drehachse 5 angeordnet ist. In der Ruheposition R der Steuerungseinrichtung 4 kann die Drehachse 5 zwischen dem Kraftübertragungselement 11 und der Halteeinrichtung 14 angeordnet sein.

Der Kraftvektor 18 der auf das Kraftübertragungselement 14 einwirkenden und durch den Kraftspeicher 12 hervorgerufenen Kraft weist vom Kraftübertragungselement 11 in Richtung auf die Halteinrichtung 14. Die durch den Kraftspeicher 12 erzeugte Kraft ist also eine Zugkraft.

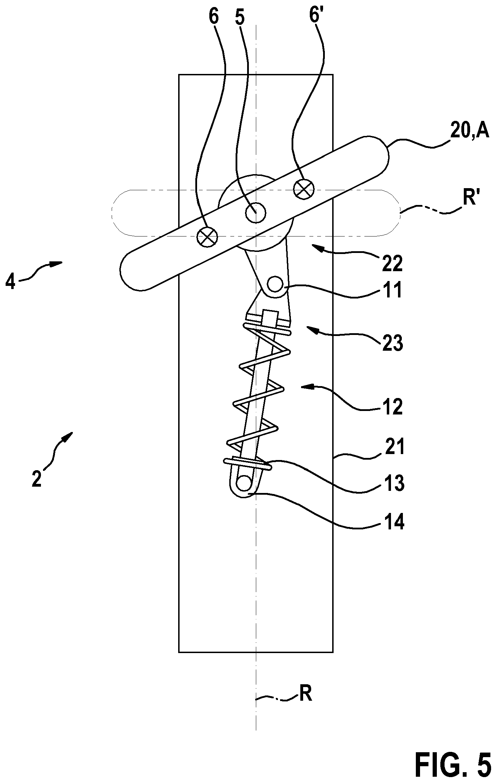

Figur 5 zeigt ein Ausführungsbeispiel der Steuerungsvorrichtung 2 des Ausführungsbeispiels der Figur 3 schematisch in einer Aufsicht. Für Elemente, die in Funktion und/oder Aufbau den Elementen des Ausführungsbeispiels der Figur 3 entsprechen, sind dieselben Bezugszeichen verwendet. Der Kürze halber ist im Folgenden lediglich auf die Unterschiede zum Ausführungsbeispiel der Figur 3 eingegangen.

Die Steuerungseinrichtung 4 ist mit einem Bedienhebel 20 ausgestaltet, der über die Drehachse 5 drehbar in einem Gehäuse 21 gehalten ist. Das Gehäuse 21 ist zumindest teilweise transparent dargestellt, um Details des Ausführungsbeispiels der Figur 5 darstellen zu können.

Das Kraftübertragungselement 11 ist als ein sich von der Drehachse 5 weg erstreckender Hebel eines um die Drehachse 5 drehbaren Exzenters 22 ausgebildet. Der Exzenter 22 ist bewegungsübertragend mit den Bedienhebel 20 verbunden und mit diesem beispielsweise verschraubt. Das Halteende 13 des Kraftspeichers 12 ist durch die Halteeinrichtung 14 im Gehäuse 21 unverschieblich, jedoch um eine sich parallel zur Drehachse 5 erstreckende Drehachse schwenkbar gehalten. Ein dem Halteende 13 gegenüberliegend angeordnetes Übertragungsende 23 des Kraftspeichers 12 ist bewegungsübertragend am Kraftübertragungselement 11 fixiert, wobei das Übertragungsende 23 um eine weitere, sich parallel zur Drehachse 5 erstreckende Drehachse relativ zum Kraftübertragungselement 11 schwenkbar ist.

Die Steuerungseinrichtung 4 ist in der Auslenkstellung A gezeigt. Die Ruhestellung R' der Steuerungseinrichtung 4 ist durch die gestrichelte Darstellung des Bedienhebels 20 gezeigt, wobei sich der Bedienhebel 20 in der Ruhestellung R' senkrecht zur gestrichelten Linie R erstreckt. Das Kraftübertragungselement 12 verläuft in der Ruhestellung R' von der Halteeinrichtung 14 in Richtung auf die Drehachse 5 entlang der gestrichelten Linie R.

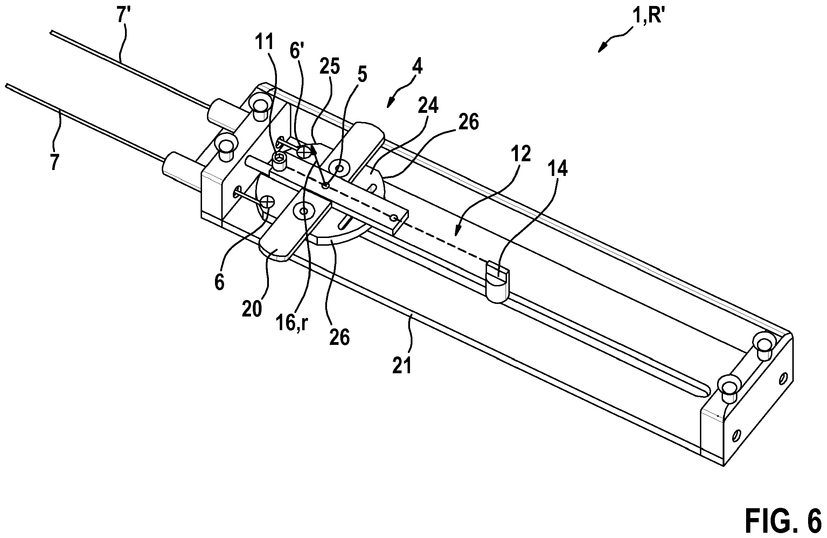

Figur 6 zeigt ein weiteres Ausführungsbeispiel der Katheterisierungsvorrichtung 1 schematisch in einer Perspektivansicht. Für Elemente, die in Funktion und/oder Aufbau den Elementen der bisherigen Ausführungsbeispiele entsprechen, sind dieselben Bezugszeichen verwendet. Der Kürze halber ist lediglich auf die Unterschiede zu den bisherigen Ausführungsbeispielen eingegangen.

Die Steuerungseinrichtung 4 ist im Ausführungsbeispiel der Figur 6 in der Ruhestellung R' dargestellt. Auch die in der Figur 6 gezeigte Katheterisierungsvorrichtung 1 ist mit dem Bedienhebel 20 ausgebildet, der um die Drehachse 5 im Gehäuse 21 ausgebildet ist. Das Gehäuse 21 ist wiederum teilweise transparent dargestellt, um Details der Katheterisierungsvorrichtung 1 besser darstellen zu können.

Der Kraftvektor 18 der durch den Kraftspeicher 12 bereitgestellten Kraft weist auf die Halteeinrichtung 14 und stellt eine Zugkraft dar, die das Kraftübertragungselement 11 in Richtung auf die Halteeinrichtung 14 zieht. Das Kraftübertragungselement 11 ist von der Halteeinrichtung 14 aus hinter der Drehachse 5 und darüber hinaus hinter dem Bedienhebel 20 angeordnet.

Das Kraftübertragungselement 11 ist an einer scheibenförmigen Basis 24 befestigt, die das Kraftübertragungselement 11, den Bedienhebel 20 und auch die Lenkdrähte 7, 7' bewegungsübertragend miteinander verbindet. Die scheibenförmige Basis 24 ist vorzugsweise unverschieblich, aber um eine Drehachse drehbar in der Katheterisierungseinrichtung 1 aufgenommen und gehalten. Zur Befestigung der Lenkdrähte 7, 7' sind die Führungselemente 6, 6' von der Halteeinrichtung 14 aus gesehen hinter dem Bedienhebel 20 und vor dem Kraftübertragungselement 11 an der Basis 23 vorgesehen, wobei die Führungselemente 6, 6' vorzugsweise zwischen den Drehachse 5 und dem Kraftübertragungselement 11 angeordnet sind.

Die Lenkdrähte 7, 7' können jeweils so an der Halteeinrichtung 14 und insbesondere an deren scheibenförmiger Basis 24 befestigt sein, dass zumindest einer der Lenkdrähte 7, 7' bei einem Auslenken der Basis 24 über diese Streicht, sodass eine senkrecht zum Kraftspeicher 12 verlaufende Komponente des Lastarms 16 ihre Länge ändert, wenn die Halteeinrichtung 14 aus der Ruheposition R' heraus bewegt wird. Die Komponente kann ebenfalls als Wirklänge des Lastarms 16 bezeichnet werden.

Im Ausführungsbeispiel der Figur 6 ist die Basis 24 jedoch mit wenigstens einer Umfangsfläche 25 ausgebildet, an der einer der Lenkdrähte 7, 7' anliegt und vorzugsweise geführt wird, wenn die Steuerungseinrichtung 4 aus der Ruheposition R' ausgelenkt ist. Weist die Umfangsfläche 25 einen konstanten Abstand zur Drehachse 5 auf, der beispielsweise dem Radius r der Umfangsfläche 25 im Bereich eines Führungsabschnittes 26 der Steuerungseinrichtung 4 zum Führen des Lenkdrahtes 7, 7' entspricht, ist die Wirklänge des Lastarms 16 unabhängig von der Auslenkung der Halteeinrichtung 14 und zum Beispiel konstant.

## Patentansprüche

1. Katheterisierungsvorrichtung (1) mit einem ein auslenkbares Lenkende aufweisenden Katheter (3) und einer Steuerungsvorrichtung (2) zur Steuerung des Lenkendes, wobei die Steuerungsvorrichtung (2) mit einer um eine Drehachse (5) drehbar gelagerten Steuerungseinrichtung (4), die zumindest ein von der Drehachse (5) beabstandet angeordnetes und bewegungsübertragend mit dem Lenkende verbundenes Führungselement (6, 6') aufweist, ausgebildet ist, weiter aufweisend einen Kraftspeicher (12), der eine eine Drehung der Steuerungseinrichtung (4) unterstützende Hilfskraft (H) bewirkt, wobei die Steuerungseinrichtung (4) ein von der Drehachse (5) und dem Führungselement (6, 6') beabstandet angeordnetes Kraftübertragungselement (11) aufweist, das Kraft übertragend mit dem Kraftspeicher (12) gekoppelt ist, **dadurch gekennzeichnet, dass** ein Lenkdraht (7, 7') an einer scheibenförmigen Basis (24) der Steuereinrichtung (4) befestigt ist.

2. Katheterisierungsvorrichtung (1) nach Anspruch 1, **dadurch gekennzeichnet, dass** ein die Drehachse (5) und das Kraftübertragungselement (11) miteinander verbindender Radius (17) senkrecht zu einem Wirkkraftvektor (19) der Hilfskraft (H) ausgerichtet ist, wobei eine Wirklänge (W) des Wirkkraftvektors (19) abhängig von einer Auslenkung der Steuerungseinrichtung (4) aus einer Ruhestellung (R') ist.

3. Katheterisierungsvorrichtung (1) nach Anspruch 2, **dadurch gekennzeichnet, dass** die Wirklänge (W) in der Ruhestellung (R') minimal ist.

4. Katheterisierungsvorrichtung (1) nach Anspruch 2 oder 3, **dadurch gekennzeichnet, dass** die Wirklänge (W) mit der Auslenkung der Steuerungseinrichtung (4) aus der Ruhestellung (R') ansteigt.

5. Katheterisierungsvorrichtung (1) nach einem der Ansprüche 2 bis 4, **dadurch gekennzeichnet, dass** der Wirkkraftvektor (19) eine Komponente der vom Kraftspeicher (12) in das Kraftübertragungselement (11) eingeleiteten Kraft ist, wobei die in das Kraftübertragungselement (11) eingeleitete Kraft in der Ruhestellung (R') der Steuerungseinrichtung (4) auf die Drehachse (5) gerichtet ist.

6. Katheterisierungsvorrichtung (1) nach einem der Ansprüche 1 bis 5, **dadurch gekennzeichnet, dass** die Steuerungsvorrichtung (2) eine Halteeinrichtung (14) für den Kraftspeicher (12) aufweist, an der der Kraftspeicher (12) zumindest in Richtung auf die Drehachse (5) unverschiebbar gehalten ist.

7. Katheterisierungsvorrichtung (1) nach einem der Ansprüche 2 bis 6, **dadurch gekennzeichnet, dass** die durch den Kraftspeicher (12) in der Ruhestellung (R') auf das Kraftübertragungselement (11) gerichtete Kraft eine Zugkraft ist, und das Kraftübertragungselement (11) in der Ruhestellung (R') zwischen der Drehachse (5) und der Halteeinrichtung (14) angeordnet ist.

8. Katheterisierungsvorrichtung (1) nach Anspruch 7, **dadurch gekennzeichnet, dass** der Kraftspeicher (12) eine Zugfeder ist.

9. Katheterisierungsvorrichtung (1) nach einem der Ansprüche 2 bis 6, **dadurch gekennzeichnet, dass** die durch den Kraftspeicher (12) in der Ruhestellung (R') auf das Kraftübertragungselement (11) gerichtete Kraft eine Druckkraft ist und die Drehachse (5) in der Ruhestellung (R') zwischen dem Kraftübertragungselement (11) und der Halteeinrichtung (14) angeordnet ist.

10. Katheterisierungsvorrichtung (1) nach Anspruch 9, **dadurch gekennzeichnet, dass** der Kraftspeicher (12) eine Druckfeder ist.

11. Katheterisierungsvorrichtung (1) nach einem der Ansprüche 1 bis 10, **gekennzeichnet durch** ein Getriebe, das den Kraftspeicher (12) Kraft übertragend mit dem Kraftübertragungselement (11) verbindet.

12. Katheterisierungsvorrichtung (1) nach einem der Ansprüche 1 bis 11, wobei der Lenkdraht (7, 7') an einer Umfangsfläche der Steuerungseinrichtung (4) geführt ist.

13. Katheterisierungsvorrichtung (1) nach einem der Ansprüche 2 bis 12, **dadurch gekennzeichnet, dass** eine Länge eines um die Drehachse (5) schwenkbaren Lastarms (16) der Steuerungseinrichtung (4) unabhängig von der Auslenkung der Steuerungseinrichtung (4) aus der Ruhestellung (R') ist.

14. Katheterisierungsvorrichtung (1) nach einem der Ansprüche 1 bis 13, **dadurch gekennzeichnet, dass** die Hilfskraft bemessen ist, das Lenkende in einer ausgelenkten Position zu halten.

15. Katheterisierungsvorrichtung (1) nach Anspruch 1, wobei die Basis (24) kreisförmig ist.

## Claims

1. A catheterisation device (1) comprising a catheter (3) having a deflectable steering end, and a control device (2) for controlling the steering end, wherein the control device (2) is formed with a control arrangement (4) which is mounted rotatably about a rotation axis (5) and which has at least one guide element (6, 6') arranged at a distance from the rotation axis (5) and connected in a movement-transmitting manner to the steering end, the control device further having an energy store (12), which produces an auxiliary force (H) assisting a rotation of the control arrangement (4), wherein the control arrangement (4) has a force transmission element (11) which is arranged at a distance from the rotation axis (5) and the guide element (6, 6') and which is coupled in a force-transmitting manner to the energy store (12), **characterised in that** a steering wire (7, 7') is secured to a planar base (24) of the control arrangement (4).

2. The catheterisation device (1) according to claim 1, **characterised in that** a radius (17) connecting the rotation axis (5) and the force transmission element (11) to one another is oriented perpendicularly to an effective-force vector (19) of the auxiliary force (H), wherein an effective length (W) of the effective-force vector (19) is dependent on a deflection of the control arrangement (4) from a rest position (R').

3. The catheterisation device (1) according to claim 2, **characterised in that** the effective length (W) in the rest position (R') is minimal.

4. The catheterisation device (1) according to claim 2 or 3, **characterised in that** the effective length (W) increases with the deflection of the control arrangement (4) from the rest position (R').

5. The catheterisation device (1) according to any one of claims 2 to 4, **characterised in that** the effective-force vector (19) is a component of the force introduced into the force transmission element (11) from the energy store (19), wherein the force introduced into the force transmission element (11) is directed towards the rotation axis (5) in the rest position (R') of the control arrangement (4).

6. The catheterisation device (1) according to any one of claims 1 to 5, **characterised in that** the control device (2) has a holding arrangement (14) for the energy store (12), on which the energy store (12) is held non-displaceably at least in the direction of the rotation axis (5).

7. The catheterisation device (1) according to any one of claims 2 to 6, **characterised in that** the force directed by the energy store (12) towards the force transmission element (11) in the rest position (R') is a tensile force, and the force transmission element (11) is arranged between the rotation axis (5) and the holding arrangement (14) in the rest position (R').

8. The catheterisation device (1) according to claim 7, **characterised in that** the energy store (12) is a tensile spring.

9. The catheterisation device (1) according to any one of claims 2 to 6, **characterised in that** the force directed by the energy store (12) towards the force transmission element (11) in the rest position (R') is a compressive force and the rotation axis (5) is arranged between the force transmission element (11) and the holding arrangement (14) in the rest position (R').

10. The catheterisation device (1) according to claim 9, **characterised in that** the energy store (12) is a compressive spring.

11. The catheterisation device (1) according to any one of claims 1 to 10, **characterised by** a gearing which connects the energy store (12) in a force-transmitting manner to the force transmission element (11).

12. The catheterisation device (1) according to any one of claims 1 to 11, wherein the steering wire (7, 7') is guided at a peripheral surface of the control arrangement (4).

13. The catheterisation device (1) according to any one of claims 2 to 12, **characterised in that** a length of a load arm (16) of the control arrangement (4) pivotable about the rotation axis (5) is independent of the deflection of the control arrangement (4) from the rest position (R').

14. The catheterisation device (1) according to any one of claims 1 to 13, **characterised in that** the auxiliary force is sufficient to hold the steering end in a deflected position.

15. The catheterisation device (1) according to claim 1, wherein the base (24) is circular.

## Revendications

1. Dispositif de cathétérisme (1) doté d'un cathéter (3) présentant une extrémité de guidage pouvant être déviée et d'un dispositif de commande (2) pour la commande de l'extrémité de guidage, où le dispositif de commande (2) est constitué d'un équipement de commande (4), logé en pouvant être mis en rotation autour d'un axe de rotation (5), qui présente au moins un élément de guidage (6, 6') disposé espacé par rapport à l'axe de rotation (5) et relié avec l'extrémité de guidage en transmettant le mouvement, présentant en outre un accumulateur de force (12) qui procure une force auxiliaire (H) apportant assistance à une rotation de l'équipement de commande (4), où l'équipement de guidage (4) présente un élément de transmission de force (11), disposé espacé par rapport à l'axe de rotation (5) et à l'élément de guidage (6, 6'), qui est couplé avec l'accumulateur de force (12) en transmettant la force, **caractérisé en ce qu'**un fil de guidage (7, 7') est fixé à une base (24) en forme de disque de l'équipement de commande (4).

2. Dispositif de cathétérisme (1) selon la revendication 1, **caractérisé en ce qu'**un rayon reliant ensemble l'axe de rotation (5) et l'élément de transmission de force (11) est orienté verticalement par rapport à un vecteur de force efficace (19) de la force auxiliaire (H), où une longueur efficace (W) du vecteur de force efficace (19) est dépendante d'une déviation de l'équipement de commande (4) à partir d'une position de repos (R').

3. Dispositif de cathétérisme (1) selon la revendication 2, **caractérisé en ce que** la longueur efficace (W) est minimale dans la position de repos (R').

4. Dispositif de cathétérisme (1) selon la revendication 2 ou la revendication 3, **caractérisé en ce que** la longueur efficace (W) augmente avec la déviation de l'équipement de commande (4) à partir de la position de repos (R').

5. Dispositif de cathétérisme (1) selon l'une des revendications 2 à 4, **caractérisé en ce que** le vecteur de force efficace (19) est une composante de la force apportée par l'accumulateur de force (12) dans l'élément de transmission de force (11), où la force apportée dans l'élément de transmission de force (11) dans la position de repos (R') de l'équipement de commande (4) s'applique sur l'axe de rotation (5).

6. Dispositif de cathétérisme (1) selon l'une des revendications 1 à 5, **caractérisé en ce que** le dispositif de commande (2) présente un dispositif de support (14) pour l'accumulateur de force (12) sur lequel l'accumulateur de force (12) est maintenu en ne pouvant pas glisser, au moins en direction de l'axe de rotation (5).

7. Dispositif de cathétérisme (1) selon l'une des revendications 2 à 6, **caractérisé en ce que** la force appliquée sur l'élément de transmission de force (11) par l'accumulateur de force (12) dans la position de repos (R') est une force de traction, et l'élément de transmission de force (11) est disposé entre l'axe de rotation (5) et le dispositif de support (14) dans la position de repos (R').

8. Dispositif de cathétérisme (1) selon la revendication 7, **caractérisé en ce que** l'accumulateur de force (12) est un ressort de traction.

9. Dispositif de cathétérisme (1) selon l'une des revendications 2 à 6, **caractérisé en ce que** la force appliquée sur l'élément de transmission de force (11) par l'accumulateur de force (12) dans la position de repos (R') est une force de compression et l'axe de rotation (5) est disposé entre l'élément de transmission de force (11) et le dispositif de support (14) dans la position de repos (R').

10. Dispositif de cathétérisme (1) selon la revendication 9, **caractérisé en ce que** l'accumulateur de force (12) est un ressort de compression.

11. Dispositif de cathétérisme (1) selon l'une des revendications 1 à 10, **caractérisé par** un entraînement qui relie l'accumulateur de force (12) avec l'élément de transmission de force (11) en transmettant la force.

12. Dispositif de cathétérisme (1) selon l'une des revendications 1 à 11, dans lequel le fil de guidage (7, 7') est mené au niveau d'une surface périphérique de l'équipement de commande (4).

13. Dispositif de cathétérisme (1) selon l'une des revendications 2 à 12, **caractérisé en ce qu'**une longueur d'un bras de charge (16) de l'équipement de commande (4) pouvant pivoter autour de l'axe de rotation (5) est indépendante de la déviation de l'équipement de commande (4) à partir de la position de repos (R').

14. Dispositif de cathétérisme (1) selon l'une des revendications 1 à 13, **caractérisé en ce que** la force auxiliaire est mesurée pour maintenir l'extrémité de guidage dans une position déviée.

15. Dispositif de cathétérisme (1) selon la revendication 1, dans lequel la base (24) est en forme de cercle.
